# EUROPEAN PATENT SPECIFICATION

(11) **EP 4 428 221 B1**
(45) Date of publication and mention of the grant of the patent: **30.09.2026**
(21) Application number: 23855039.6
(22) Date of filing: 06.07.2023
(51) Int. Cl.: C12M 1/34, C12M 1/21, G01N 33/18

(54) **SOLUTION STATE MEASUREMENT DEVICE**
VORRICHTUNG ZUR MESSUNG DES LÖSUNGSZUSTANDS
DISPOSITIF DE MESURE DE L'ÉTAT D'UNE SOLUTION

(30) Priority: 16.08.2022 KR 20220102208; 30.06.2023 KR 20230085322
(43) Date of publication of application: 11.09.2024
(73) Proprietor: LG Chem, Ltd., Yeongdeungpo-gu Seoul 07336 (KR)
(72) Inventor: KWON, Youbin, Daejeon 34122 (KR); CHOI, Jaegil, Daejeon 34122 (KR); SONG, Youngsoo, Daejeon 34122 (KR); IM, Ye Hoon, Daejeon 34122 (KR); KIM, Jaehyun, Daejeon 34122 (KR); CHOI, Ikjin, Daejeon 34122 (KR); NAM, Gibok, Daejeon 34122 (KR); SEON, Gyeongho, Daejeon 34122 (KR)
(74) Representative: Hoffmann Eitle
(86) International application number: PCT/KR2023/009556
(87) International publication number: WO 2024/039068

(56) References cited:
- EP-A1- 3 819 629
- JP-A- 2006 317 285
- JP-U- S62 192 242
- KR-A- 20110 040 356
- KR-A- 20140 139 656
- KR-B1- 101 746 878
- KR-B1- 101 887 472
- KR-B1- 101 887 472
- US-B2- 8 988 082

## Description

### [Technical Field]

### CROSS-REFERENCE TO RELATED APPLICATION

This application claims priority to and the benefit of Korean Patent Application No. 10-2022-0102208 filed in the Korean Intellectual Property Office on August 16, 2022.

The present invention relates to a device for detecting a solution state, and more particularly, to a device for detecting a solution state which includes a mesh disposed in a path through which a solution is introduced into a probe for detecting the solution state to prevent a bubble from entering a detecting portion of the probe, thereby preventing an error from occurring in the detected solution state.

### [Background Art]

3-Hydropropionic Acid (3-HP) may be a platform compound that may be converted into many other useful chemicals, including acrylic acid, and thus may be widely applied to production of pigments, paints, fibers, or the like. The 3-HP may be a representative biodegradable material that may be produced by fermenting colon bacillus.

The colon bacillus may be cultured and fermented in a culture fluid in a reactor. A spectrometer may be installed in this reactor to detect a concentration of the colon bacillus in the culture fluid in real time. In addition, various detection devices may be installed in the reactor to detect a state (e.g., concentration, capacitance, or inductance) of the culture fluid, thereby predicting a state of the colon bacillus. These detection devices may be typically configured to detect the state of the culture fluid flowing into the detecting portion in real time.

An agitating device may also be disposed in the reactor for cultivating the colon bacillus. The agitating device may be configured to agitate the culture fluid and supply oxygen to the colon bacillus in the culture fluid. When the agitating device is operated and agitates the culture fluid, a bubble or the like may occur in the culture fluid, and the occurring bubble may be introduced into the detecting portion of the detection device along with the culture fluid. As such, when the bubble is present in or passes through the detecting portion, the state of the culture fluid detected by the detection device may not be uniform. That is, an error may occur in the state of the culture fluid detected by the detection device.

EP 3 819 629 A1 relates to a cover for a sensor used to measure properties of a solution into which air is mixed, and a sensor apparatus including same.

JP S62 192242 U relates to a turbidity sensor that measures suspended solids concentration and bacterial cell concentration using turbidity, and in particular to a turbidity sensor that eliminates measurement errors caused by air bubbles.

The above information disclosed in this Background section is provided only to assist in more understanding of the background of the present disclosure, and may thus include information not included in the prior art already known to those skilled in the art to which the present disclosure pertains.

### [Disclosure]

### [Technical Problem]

The present invention attempts to provide a device for detecting a solution state which includes a mesh disposed in a path through which a solution is introduced into a probe for detecting the solution state to prevent a bubble from entering the probe.

### [Technical Solution]

According to the present invention, provided is a device for detecting a solution state, the device including: a probe including a detecting portion having at least one open surface to detect the solution state; a mesh disposed on a path through which a solution is introduced into the detecting portion; and a support portion disposed on an outside of the probe, and having an opening surrounding the at least one open surface of the detecting portion, wherein the opening defines the path through which the solution is introduced into the detecting portion, and the mesh is mounted in the opening of the support portion.

An inner surface of the support portion may be spaced apart from an outer surface of the probe by a predetermined distance.

The support portion may be detachably mounted on an outer surface of the probe through a fastening portion.

According to the present invention, the support portion includes first and second support portions spaced apart from each other by a predetermined distance, and the first and second support portions are detachably assembled to each other for the mesh to be mounted at the predetermined distance between the first and second support portions.

The first and second support portions may be screwed to each other.

The second support portion may be inserted into the first support portion while being spaced apart from the first support portion by the predetermined distance, and a first thread portion may be formed on an inner surface of a lower portion of the first support portion, a second thread portion may be formed on an outer surface of a lower portion of the second support portion, and the first thread portion and the second thread portion may be screwed to each other.

Upper ends of the first and second support portions may be open.

A lower end of the first support portion may be open, and a lower end of the second support portion may be open, closed, or openable.

An inner surface of the second support portion and an outer surface of the probe may be spaced apart from each other by the predetermined distance.

A fastening portion may be formed in an upper portion of the first support portion, and the support portion may be detachably mounted on an outer surface of the probe through the fastening portion.

The fastening portion may be disposed above an upper end of the second support portion.

### [Advantageous Effects]

According to the present invention, the mesh is disposed in the path through which the solution is introduced into the probe to prevent the bubble from entering the probe.

According to the present invention, the mesh is mounted on the support portion, and the support portion is detachably mounted on the probe. Accordingly, the mesh and the support portion are thus easily installed or replaced.

In addition, the support portion may be made larger than the probe by the predetermined size, thereby preventing the bubble from escaping out of the support portion and causing the error in the detection value of the state even if the bubble is present between the support portion and the probe.

Other effects which may be acquired or predicted by the embodiments of the present disclosure are disclosed directly or implicitly in the detailed description of the embodiments of the present disclosure. That is, various effects predicted based on the embodiments of the present disclosure are disclosed in the detailed description described below.

### [Description of the Drawings]

Embodiments in the specification may be better understood by reference to the following description in connection with the accompanying drawings in which like reference numerals refer to identical or functionally similar elements.
FIG. 1 is a perspective view of a device for detecting a solution state according to an embodiment of the present disclosure.
FIG. 2 is a perspective view of a probe according to an embodiment of the present disclosure.
FIG. 3 is a perspective view of a support portion according to an embodiment of the present disclosure.
FIG. 4 is a perspective view of a first support portion included in the support portion of FIG. 3.
FIG. 5 is a perspective view of a second support portion included in the support portion of FIG. 3.
FIG. 6 shows a spectrum depending on the presence or absence of a bubble in the probe.
FIG. 7 shows an example of a spectrum of a culture fluid detected using a device for detecting a solution state according to an embodiment of the present disclosure.
FIG. 8 shows an example of a spectrum of a culture fluid detected using a device for detecting a solution state according to the prior art.

It should be understood that the drawings referenced above are not necessarily drawn to scale, and present a rather simplified representation of various preferred features showing the basic principles of the present disclosure. For example, specific design features of the present disclosure, including its specific dimension, orientation, position and shape, are determined in part by the particular intended application and environment of use.

### [Mode for Invention]

A term used herein is only to describe a specific embodiment, and is not intended to limit the present disclosure. A term of a singular number used herein is intended to include its plural number unless the context clearly indicates otherwise. It should also be understood that the terms "include" and/or "including," when used in the specification, specify the presence of the recited features, integers, steps, operations, elements and/or components, and do not exclude the presence or addition of one or more of other features, integers, steps, operations, elements, components and/or groups thereof. A term "and/or" used herein includes any one or all combinations of the associated listed items.

A device for detecting a solution state according to the present invention includes a mesh disposed in a path through which a solution is introduced into a probe to prevent a bubble from entering the probe. Accordingly, it is possible to accurately detect the solution state. In addition, the mesh is mounted on a support portion, and the support portion may be detachably mounted on the probe. Accordingly, the mesh and the support portion are thus easily installed or replaced. In addition, the support portion may be made larger than the probe by a predetermined size, thereby preventing the bubble from escaping out of the support portion and causing an error in a detection value of the state even if the bubble is present between the support portion and the probe.

Hereinafter, an embodiment of the present disclosure is described in detail with reference to the accompanying drawings.

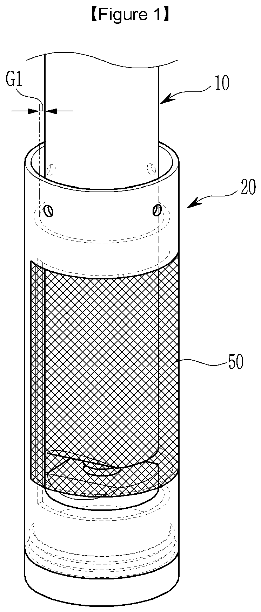

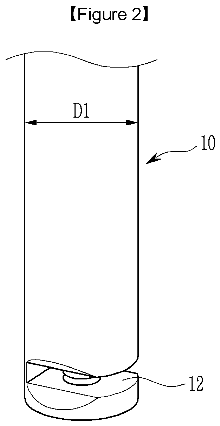

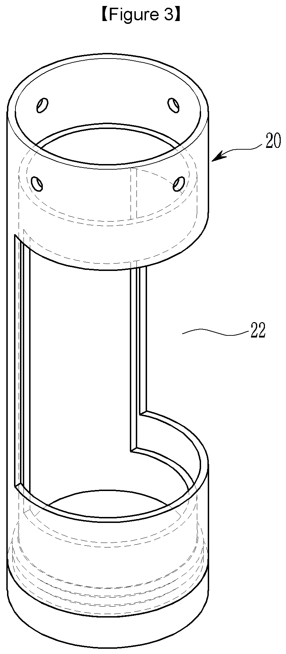

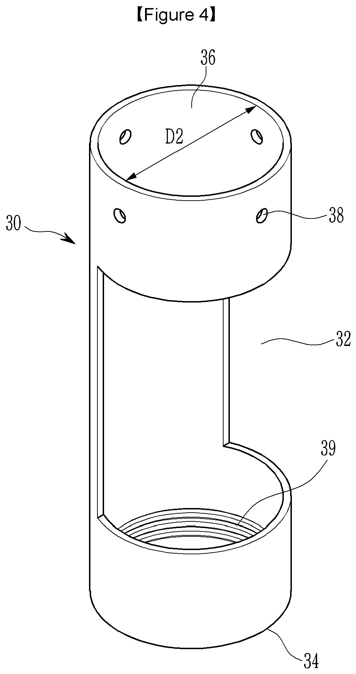

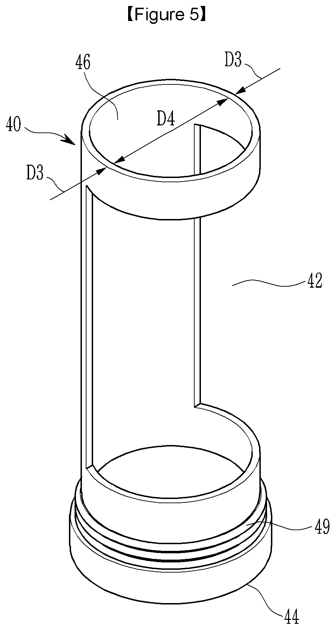

FIG. 1 is a perspective view of a device for detecting a solution state according to an embodiment of the present disclosure; FIG. 2 is a perspective view of a probe according to an embodiment of the present disclosure; FIG. 3 is a perspective view of a support portion according to an embodiment of the present disclosure; FIG. 4 is a perspective view of a first support portion included in the support portion of FIG. 3; and FIG. 5 is a perspective view of a second support portion included in the support portion of FIG. 3.

As shown in FIGS. 1 to 5, a device for detecting a solution state according to an embodiment of the present disclosure may include a probe 10, a support portion 20, and a mesh 50.

As shown in FIGS. 1 and 2, the probe 10 is configured to detect the solution state (e.g., a concentration, a capacitance, or a inductance of a solution). The probe 10 may have a pillar shape, and have an upper portion fixed to or passing through an upper portion or a lid of a reactor, and a lower portion immersed in the solution in the reactor. For example, a detecting portion 12 may be disposed in the lower portion of the probe 10, and detect the state of the solution which is present in or passes through the detecting portion 12. For this purpose, the lower portion of the probe 10, especially at least the detecting portion 12, may be disposed to be immersed in the solution. In addition, at least one surface of the detecting portion 12 may be open and function as an inlet through which the solution is introduced into the detecting portion 12 or an outlet through which the solution escapes from the detecting portion 12. In this specification, it is exemplified that the probe 10 has a circular column shape and the support portion 20 has a cylinder shape. However, the respective shapes of the probe 10 and the support portion 20 are not limited to the circular column shape and the cylinder shape. When the probe 10 has the circular column shape, the probe 10 may be shown as having a diameter of D1.

As shown in FIGS. 1 and 3 and according to the present invention, the support portion 20 is provided to mount the mesh 50 around the detecting portion 12, and detachably mounted on the probe 10. When the probe 10 has the circular column shape, the support portion 20 may have a hollow cylinder shape, and surround the probe 10. The support portion 20 has an opening 22 formed therein. The opening 22 may surround a part of an outer peripheral surface of the probe 10, for example, at least the open surface of the detecting portion 12. Therefore, the solution in the reactor may be introduced into the detecting portion 12 only through the opening 22. That is, the opening 22 forms a path through which the solution is introduced into the detecting portion 12 of the probe 10.

According to the present invention, the support portion 20 is provided as two or more items and are detachably assembled to each other. According to the present invention, the support portion 20 includes a first support portion 30 and a second support portion 40. Here, the description exemplifies that the number of items included in the support portion 20 is two. However, it should be understood that the number of items included in the support portion 20 is not necessarily limited to two.

As shown in FIGS. 1, 3 and 4, the first support portion 30 may correspond to an outer shell of the support portion 20. The first support portion 30 may include a first opening 32 corresponding to the opening 22. In addition, the first support portion 30 includes a lower end 34 and an upper end 36. The lower end 34 of the first support portion 30 may be open so that the second support portion 40 may be inserted into the first support portion 30 from bottom to top. Similarly, the upper end 36 of the first support portion 30 may be open so that the probe 10 may be inserted into the first support portion 30 from top to bottom, that is, so that the first support portion 30 may be moved from bottom to top to a mounting position of the first support portion 30 on the probe 10. When the first support portion 30 has the hollow cylinder shape, the first support portion 30 may be shown as having an inner diameter of D2.

According to the present invention, the first support portion 30 is detachably assembled to the second support portion 40. In an example, a first thread portion 39 may be formed on a lower portion of an inner peripheral surface of the first support portion 30, and may be screwed to the second support portion 40. In addition, the first support portion 30 may be detachably mounted on the outer peripheral surface of the probe 10. For this purpose, a fastening portion 38 may be formed in the upper portion of the first support portion 30. In an example, the fastening portion 38 may be a fastening hole. In this case, a corresponding fastening hole may be formed in the outer peripheral surface of the probe 10. Accordingly, the first support portion 30 may be mounted on the outer peripheral surface of the probe 10 by inserting a screw, a pin, or the like into the fastening hole of the first support portion 30 and the corresponding fastening hole of the probe 10. In addition, the first support portion 30 may be detached from the probe 10 by removing the screw, the pin, or the like from the fastening hole and/or the corresponding fastening hole. Here, the description exemplifies that the fastening portion 38 is the fastening hole, and the present disclosure is not limited thereto.

As shown in FIGS. 1, 3 and 5, the second support portion 40 may correspond to an inner shell of the support portion 20. The second support portion 40 may include a second opening 42 corresponding to the opening 22. Therefore, when the first support portion 30 and the second support portion 40 are assembled to each other, the first opening 32 and the second opening 42 are coupled to each other to thus form the opening 22.

The second support portion 40 may include a lower end 44 and an upper end 46. The lower end 44 of the second support portion 40 may be open, closed, or openable by a lid, or the like. In general, the bubble occurring by an agitating device may have a low specific gravity to float on top of the solution. Therefore, the bubble may not be introduced to the detecting portion 12 through the open lower end 44 of the second support portion 40. Therefore, even when the lower end 44 of the second support portion 40 is open, the open lower end may have little effect on an operation of the detecting portion 12. The upper end 46 of the second support portion 40 may be open so that the probe 10 may be inserted into the second support portion 40 from top to bottom, that is, so that the second support portion 40 may be moved from bottom to top to a mounting position of the support portion 40 on the probe 10. When the second support portion 40 has the hollow cylinder shape, the second support portion 40 may be shown as having an outer diameter of D3 and the second support portion 40 may be shown as having an inner diameter of D4.

As described above, the second support portion 40 is detachably assembled to the first support portion 30. In an example, a second thread portion 49 may be formed on a lower portion of an outer peripheral surface of the second support portion 40, and may be screwed to the first thread portion 39 of the first support portion 30. In addition, the upper end 46 of the second support portion 40 may be disposed below the fastening portion 38 so that the support portion 20 formed by coupling the first support portion 30 with the second support portion 40, may be detachably mounted on the outer peripheral surface of the probe 10. That is, the fastening portion 38 may be disposed above the upper end 46 of the second support portion 40. Therefore, the support portion 20 may be detachably mounted on the outer peripheral surface of the probe 10 by using only the fastening portion 38 without forming a fastening portion corresponding to the fastening portion 38 in the second support portion 40.

According to the present invention, , the inner diameter D2 or the inner surface of the first support portion 30 is spaced apart from the outer diameter D3 or the outer surface of the second support portion 40 by a predetermined distance. The predetermined distance is provided to install the mesh 50 between the inner diameter D2 or the inner surface of the first support portion 30 and the outer diameter D3 or the outer surface of the second support portion 40. The predetermined distance is not limited thereto, and may range from 0.3 mm to 1mm. In addition, the outer diameter D1 or the outer surface of the probe 10 and the inner diameter D4 or the inner surface of the second support portion 40 may be spaced apart from each other by the predetermined distance G1. Even if the bubble is present or introduced between the probe 10 and the support portion 20, the bubble may escape through the predetermined distance G1. The predetermined distance G1 is not limited thereto, and may range from 2 mm to 5 mm.

According to the present invention, the mesh 50 is mounted in the opening 22 surrounding the open surface of the detecting portion 12 of the probe 10. That is, the mesh 50 is disposed on the path through which the solution is introduced into the detecting portion 12 of the probe 10, thus preventing the bubble in the solution from being introduced into the detecting portion 12 of the probe 10. As described above, the mesh 50 is mounted at the predetermined distance between the inner diameter D2 or the inner surface of the first support portion 30 and the outer diameter D3 or the outer surface of the second support portion 40. In addition, the lower end of the mesh 50 may not deviate from the predetermined distance by being supported by the first and second thread portions 39 and 49 screwed to each other. That is, the mesh 50 may deviate only upward from the predetermined distance, which means that the mesh 50 does not deviate from the support portion 20 without a user's intention to detach the mesh 50. The mesh 50 is not limited to any particular size as long as the mesh has a large size enough to prevent the bubble from being introduced into the detecting portion 12. In an example, the mesh 50 could be a 300 mesh or a 500 mesh. In another example, the mesh 50 may have a size determined based on the size, flow rate, or the like of the bubble occurring in an operation condition of the reactor. For example, the mesh 50 may be 100 mesh to 1000 mesh.

A material of the mesh 50 is not particularly limited, and the mesh 50 may be used as long as the mesh 50 is made of a material that does not corrode in a liquid. In an example, the material of the mesh 50 may be a metal material such as a steel use stainless (SUS) material or a plastic material, which has a strong corrosion resistance.

In addition, a hole in the mesh 50 is not limited to any particular shape as long as the mesh 50 may prevent passage of the bubble and reduce its flow rate. In an example, the hole may have various shapes, such as a circle or a polygon.

Hereinafter, the description briefly describes a process of installing the device for detecting a solution state on the probe 10. In an example, the probe 10 may have the circular column shape, and may be configured to include the upper portion fixed to or passing through the upper portion or lid of the reactor and the lower portion immersed in the solution in the reactor.

First, the support portion 20 may be formed by screwing the first and second thread portions 39 and 49 to each other and assembling the first and second support portions 30 and 40 to each other. Here, the opening 22 may be formed by coupling the first and second openings 32 and 42 to each other. In addition, the upper end 46 of the second support portion 40 may be disposed below the fastening portion 38, and the inner diameter D2 of the first support portion 30 and the outer diameter D3 of the second support portion 40 may be spaced apart from each other by the predetermined distance.

In this state, the mesh 50 is inserted downward into the predetermined distance between the inner diameter D2 of the first support portion 30 and the outer diameter D3 of the second support portion 40. The mesh 50 is mounted in the opening 22, which is the path through which the solution is introduced into the detecting portion 12 of the probe 10.

The support portion 20 equipped with the mesh 50 may then be mounted on the outer peripheral surface of the probe 10 through the fastening portion 38. Here, the mesh 50 may surround the open surface of the detecting portion 12, and the outer diameter D1 of the probe 10 and the inner diameter D4 of the second support portion 40 may be spaced apart from each other by the predetermined distance G1, thus forming a space for the bubble to escape between the probe 10 and the support portion 20.

Hereinafter, the description describes an effect of the device for detecting a solution state according to an embodiment of the present disclosure.

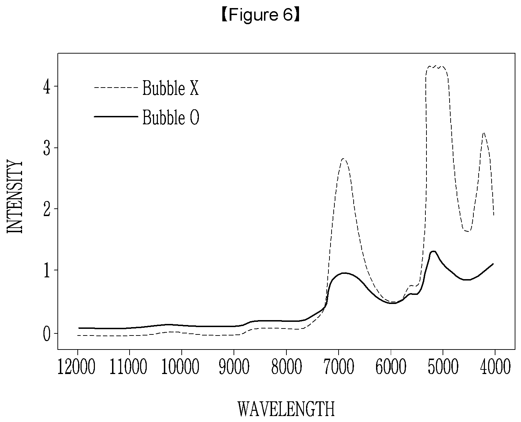

FIG. 6 shows a spectrum depending on the presence or absence of the bubble in the probe. FIG. 6 shows the spectrum for the cases where the bubble is present and absent in the detecting portion 12 of the probe 10 when the reactor is stopped. In FIG. 6, a dotted line represents the spectrum when the bubble is absent in the detecting portion 12, and a solid line represents the spectrum when bubble is present in the detecting portion 12.

As may be seen from the dotted line in FIG. 6, when the bubble is absent in the detecting portion 12, a peak having an intensity of 7000 Å, 5000 Å, or 4000 Å may be generated. However, as may be seen from the solid line in FIG. 6, when the bubble is present in the detecting portion 12, the bubble may cause the error in the detection value of the detecting portion 12, and the peak may not be generated or may have a low intensity even when the peak is generated.

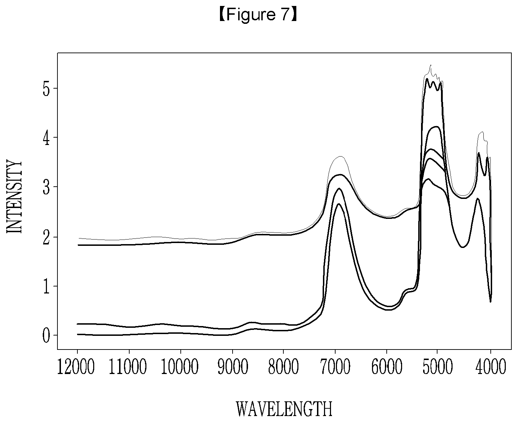

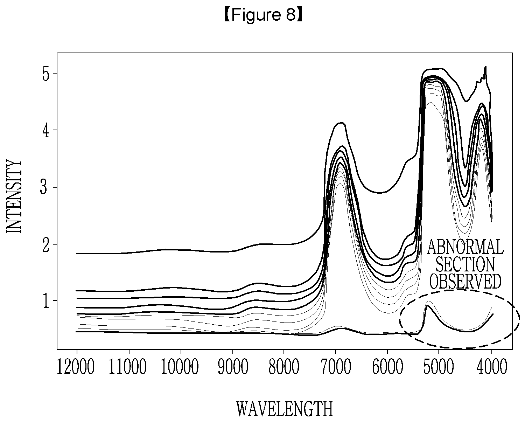

FIG. 7 shows an example of a spectrum of the culture fluid detected using the device for detecting a solution state according to an embodiment of the present disclosure; and FIG. 8 shows an example of a spectrum of a culture fluid detected using a device for detecting a solution state according to the prior art. FIGS. 7 and 8 show a change in the spectrum that is detected in a process of cultivating the colon bacillus for 24 hours.

As shown in FIG. 7, it may be seen that the spectrum indicated by the dotted line in FIG. 6 is generated when the spectrum of the culture fluid is detected using the device for detecting a solution state according to an embodiment of the present disclosure. That is, the device for detecting a solution state according to an embodiment of the present disclosure may prevent the bubble from being introduced to the detecting portion 12, thus acquiring an accurate spectrum.

As shown in FIG. 8, an abnormal section may be observed at a low wavelength when detecting the spectrum of the culture fluid using the device for detecting a solution state according to the prior art. That is, the device for detecting a solution state according to the prior art may not have a means to prevent the bubble from being introduced to the detecting portion, and an error may occur in the detection value.

## Claims

1. A device for detecting a solution state, the device comprising:
a probe (10) including a detecting portion (12) having at least one open surface to detect the solution state;
a mesh (50) disposed on a path through which a solution is introduced into the detecting portion (12); and
a support portion (20) disposed on an outside of the probe (10), and having an opening (22) surrounding the at least one open surface of the detecting portion (12),
wherein the opening (22) defines the path through which the solution is introduced into the detecting portion (12), and
the mesh (50) is mounted in the opening (22) of the support portion (20),
**characterized in that**:
the support portion (20) includes first and second support portions (30, 40) spaced apart from each other by a predetermined distance, and
the first and second support portions (30, 40) are detachably assembled to each other for the mesh (50) to be mounted at the predetermined distance between the first and second support portions (30, 40).

2. The device of claim 1, wherein
an inner surface of the support portion (20) is spaced apart from an outer surface of the probe (10) by a predetermined distance.

3. The device of claim 1, wherein
the support portion (20) is detachably mounted on an outer surface of the probe (10) through a fastening portion (38).

4. The device of claim 1, wherein
the first and second support portions (30, 40) are screwed to each other.

5. The device of claim 4, wherein
the second support portion (40) is inserted into the first support portion (30) while being spaced apart from the first support portion (30) by the predetermined distance, and
a first thread portion (39) is formed on an inner surface of a lower portion of the first support portion (30), a second thread portion (49) is formed on an outer surface of a lower portion of the second support portion (40), and the first thread portion (39) and the second thread portion (49) are screwed to each other.

6. The device of claim 1, wherein
upper ends of the first and second support portions (30, 40) are open.

7. The device of claim 1, wherein
a lower end of the first support portion (30) is open, and
a lower end of the second support portion (40) is open, closed, or openable.

8. The device of claim 1, wherein
an inner surface of the second support portion (40) and an outer surface of the probe (10) are spaced apart from each other by the predetermined distance.

9. The device of claim 1, wherein
a fastening portion (38) is formed in an upper portion of the first support portion (30), and
the support portion (20) is detachably mounted on an outer surface of the probe (10) through the fastening portion (38).

10. The device of claim 9, wherein
the fastening portion (38) is disposed above an upper end of the second support portion (40).

## Patentansprüche

1. Vorrichtung zum Detektieren eines Lösungszustands, wobei die Vorrichtung Folgendes umfasst:
eine Sonde (10), die einen Detektionsabschnitt (12) einschließt, der mindestens eine offene Oberfläche zum Detektieren des Lösungszustands aufweist;
ein Netz (50), das auf einem Pfad angeordnet ist, durch den eine Lösung in den Detektionsabschnitt (12) eingeführt wird; und
einen Stützabschnitt (20), der an der Außenseite der Sonde (10) angeordnet ist und eine Öffnung (22) aufweist, die die mindestens eine offene Oberfläche des Detektionsabschnitts (12) umgibt,
wobei die Öffnung (22) den Pfad definiert, durch den die Lösung in den Detektionsabschnitt (12) eingeführt wird, und
das Netz (50) in der Öffnung (22) des Stützabschnitts (20) montiert ist,
**dadurch gekennzeichnet, dass**
der Stützabschnitt (20) erste und zweite Stützabschnitte (30, 40) einschließt, die durch einen vorbestimmten Abstand voneinander beabstandet sind, und
die ersten und zweiten Stützabschnitte (30, 40) lösbar miteinander verbunden sind, damit das Netz (50) in dem vorbestimmten Abstand zwischen den ersten und zweiten Stützabschnitten (30, 40) montiert ist.

2. Vorrichtung nach Anspruch 1, wobei
eine Innenfläche des Stützabschnitts (20) von einer Außenfläche der Sonde (10) durch einen vorbestimmten Abstand beabstandet ist.

3. Vorrichtung nach Anspruch 1, wobei
der Stützabschnitt (20) über einen Befestigungsabschnitt (38) lösbar an einer Außenfläche der Sonde (10) montiert ist.

4. Vorrichtung nach Anspruch 1, wobei
die ersten und zweiten Stützabschnitte (30, 40) miteinander verschraubt sind.

5. Vorrichtung nach Anspruch 4, wobei
der zweite Stützabschnitt (40) in den ersten Stützabschnitt (30) eingesetzt ist, während er von dem ersten Stützabschnitt (30) durch den vorbestimmten Abstand beabstandet ist, und
ein erster Gewindeabschnitt (39) auf einer Innenfläche eines unteren Abschnitts des ersten Stützabschnitts (30) gebildet ist, ein zweiter Gewindeabschnitt (49) auf einer Außenfläche eines unteren Abschnitts des zweiten Stützabschnitts (40) gebildet ist, und der erste Gewindeabschnitt (39) und der zweite Gewindeabschnitt (49) miteinander verschraubt sind.

6. Vorrichtung nach Anspruch 1, wobei
obere Enden der ersten und zweiten Stützabschnitte (30, 40) offen sind.

7. Vorrichtung nach Anspruch 1, wobei
ein unteres Ende des ersten Stützabschnitts (30) offen ist, und
ein unteres Ende des zweiten Stützabschnitts (40) offen, geschlossen oder öffnungsfähig ist.

8. Vorrichtung nach Anspruch 1, wobei
eine Innenfläche des zweiten Stützabschnitts (40) und eine Außenfläche der Sonde (10) durch den vorbestimmten Abstand voneinander beabstandet sind.

9. Vorrichtung nach Anspruch 1, wobei
ein Befestigungsabschnitt (38) in einem oberen Abschnitt des ersten Stützabschnitts (30) gebildet ist, und
der Stützabschnitt (20) über den Befestigungsabschnitt (38) lösbar an einer Außenfläche der Sonde (10) montiert ist.

10. Vorrichtung nach Anspruch 9, wobei
der Befestigungsabschnitt (38) oberhalb eines oberen Endes des zweiten Stützabschnitts (40) angeordnet ist.

## Revendications

1. Dispositif de détection de l'état d'une solution, le dispositif comprenant :
une sonde (10) incluant une partie de détection (12) présentant au moins une surface ouverte pour détecter l'état de la solution ;
une grille (50) disposée sur un trajet à travers lequel une solution est introduite dans la partie de détection (12) ; et
une partie de support (20) disposée à l'extérieur de la sonde (10), et présentant une ouverture (22) entourant l'au moins une surface ouverte de la partie de détection (12),
dans lequel l'ouverture (22) définit le trajet à travers lequel la solution est introduite dans la partie de détection (12), et
la grille (50) est montée dans l'ouverture (22) de la partie de support (20),
**caractérisé en ce que** :
la partie de support (20) inclut des première et deuxième parties de support (30, 40) espacées l'une de l'autre selon une distance prédéterminée, et
les première et deuxième parties de support (30, 40) sont assemblées l'une à l'autre de manière amovible pour que la grille (50) soit montée à la distance prédéterminée entre les première et deuxième parties de support (30, 40).

2. Dispositif selon la revendication 1, dans lequel
une surface interne de la partie de support (20) est espacée d'une surface externe de la sonde (10) selon une distance prédéterminée.

3. Dispositif selon la revendication 1, dans lequel
la partie de support (20) est montée de manière amovible sur une surface externe de la sonde (10) par l'intermédiaire d'une partie de fixation (38).

4. Dispositif selon la revendication 1, dans lequel
les première et deuxième parties de support (30, 40) sont vissées l'une à l'autre.

5. Dispositif selon la revendication 4, dans lequel
la deuxième partie de support (40) est insérée dans la première partie de support (30) tout en étant espacée de la première partie de support (30) selon la distance prédéterminée, et
une première partie filetée (39) est formée sur une surface interne d'une partie inférieure de la première partie de support (30), une deuxième partie filetée (49) est formée sur une surface externe d'une partie inférieure de la deuxième partie de support (40), et la première partie filetée (39) et la deuxième partie filetée (49) sont vissées l'une à l'autre.

6. Dispositif selon la revendication 1, dans lequel
des extrémités supérieures des première et deuxième parties de support (30, 40) sont ouvertes.

7. Dispositif selon la revendication 1, dans lequel
une extrémité inférieure de la première partie de support (30) est ouverte, et
une extrémité inférieure de la deuxième partie de support (40) est ouverte, fermée ou ouvrable.

8. Dispositif selon la revendication 1, dans lequel
une surface interne de la deuxième partie de support (40) et une surface externe de la sonde (10) sont espacées l'une de l'autre selon la distance prédéterminée.

9. Dispositif selon la revendication 1, dans lequel
une partie de fixation (38) est formée dans une partie supérieure de la première partie de support (30), et
la partie de support (20) est montée de manière amovible sur une surface externe de la sonde (10) par l'intermédiaire de la partie de fixation (38).

10. Dispositif selon la revendication 9, dans lequel
la partie de fixation (38) est disposée au-dessus d'une extrémité supérieure de la deuxième partie de support (40).
